Europäisches Patentamt

European Patent Office

Office européen des brevets

(11) Veröffentlichungsnummer: **0 241 595**

**A1**

(12) **EUROPÄISCHE PATENTANMELDUNG**

(21) Anmeldenummer: **86117838.2**

(22) Anmeldetag: **20.12.86**

(51) Int. Cl.⁴: **A61M 1/00 , A61M 31/00**

(30) Priorität: **01.10.86 DE 8600458 U**
**04.03.86 DE 3611112**

(43) Veröffentlichungstag der Anmeldung:
**21.10.87 Patentblatt 87/43**

(84) Benannte Vertragsstaaten:
**AT BE CH DE ES FR GB GR IT LI LU NL SE**

(71) Anmelder: **Fresenius AG**
**Gluckensteinweg 5**
**D-6380 Bad Homburg(DE)**

(72) Erfinder: **Ufermann, Rüdiger**
**Scherpenberger Strasse 111**
**D-4130 Moers(DE)**

(74) Vertreter: **Görtz, Dr. Fuchs, Dr. Luderschmidt**
**Patentanwälte**
**Sonnenberger Strasse 100 Postfach 26 26**
**D-6200 Wiesbaden(DE)**

(54) **System für enterale Ernährung.**

(57) Die Erfindung betrifft ein Ernährungssystem (1) für die enterale Ernährung, das einen vorzugsweise als Einmalartikel ausgebildeten Behälter (2) zur Aufnahme der Nahrung aufweist. Dieser Behälter (2) ist in einer Aufhängevorrichtung (3) angeordnet, die derart ausgebildet ist, daß der Behälter (2) im aufgehängten Zustand mit seiner Öffnung (5) nach oben weist und eine zur Horizontalen geneigte Lage einnimmt. Dadurch wird erreicht, daß es bei in den Behälter (2) eingeführtem Schlauchleitungssystem (4) zur Zuführung der Nährlösung zum Patienten nicht zu Undichtigkeiten kommen kann, da die Öffnung (5) nach oben weist, so daß entlang des Umfanges eines in den Behälter (2) eingeführten Einsteckrohres (17) keine Flüssigkeit heraustropfen kann. Trotzdem kann durch die geneigte Lage des Behälters (2) und die Anordnung des Einsteckrohres (17) an der tiefsten Stelle (6) des Behälters (2) sämtliche Flüssigkeit entnommen werden, wozu zum Ansaugen vorzugsweise eine Tropfkammer (21) in Verbindung mit einer Schlauchklemme (26) vorgesehen ist.

Fig.1

## Ernährungssystem für die enterale Ernährung

Die Erfindung betrifft ein Ernährungssystem für die enterale Ernährung, nach dem Oberbegriff des Anspruchs 1.

Bisher bekannte, dem Oberbegriff des Anspruchs 1 entsprechende Ernährungssysteme für die enterale Ernährung weisen einen Behälter zur Aufnahme der Nahrung auf, der eine mit einem durchstechbaren Verschluß versehene oder aufreißbare Öffnung enthält. Der Behälter wird mittels einer Aufhängevorrichtung aufgehängt und an ein Schlauchleitungssystem angeschlossen, dessen eines Ende im Behälter angeordnet ist, während das andere Ende der zu ernährenden Person zugeführt wird.

Hierbei ist jedoch nachteilig, daß als Behälter entweder Glasflaschen oder Dosen verwendet werden, in die zunächst die Nahrung eingefüllt werden muß. Danach werden die Behälter mit der Öffnung nach unten weisend aufge hängt, was praktisch immer zu Undichtigkeiten führt, so daß Nährlösung zum Teil aus dem Behälter austropft und nicht in das Schlauchsystem gelangt. Wie im Rahmen der Erfindung durchgeführte Untersuchungen gezeigt haben, hat daran auch der Versuch nichts ändern können, die Behälter seitlich zu durchstechen, da auch hierbei die unerwünschten Undichtigkeiten aufgetreten sind.

Es ist daher Aufgabe der Erfindung, ein Ernährungssystem für die enterale Ernährung der im Oberbegriff des Anspruchs 1 umrissenen Gattung zu schaffen, bei welchem auf zuverlässige Art und Weise das Verhindern von Undichtigkeiten im die Nahrung aufnehmenden Behälter möglich ist.

Diese Aufgabe wird durch die Merkmale des Anspruchs 1 gelöst.

Dadurch wird erreicht, daß Undichtigkeitsprobleme gar nicht erst auftreten können, da der Behälter derart aufgehängt ist, daß seine Öffnung nach oben weist und somit Nährflüssigkeit im Bereich um das im Behälter angeordnete Ende herum nicht aus der Öffnung austreten kann. Um sicherzustellen, daß trotz dieser Anordnung des Behälters die gesamte Nährflüssigkeit diesem entnommen werden kann, ist der Behälter in einem Winkel zur Horizontalen geneigt aufgehängt und das im Behälter angeordnete Ende des Schlauchleitungssystems, das als Einstechrohr ausgebildet ist, ist mit seinem offenen Ende an der tiefsten Stelle des Behälters angeordnet. Handelt es sich beispielsweise um einen Behälter mit rechteckiger Form, ergibt sich aus der Aufhängung, daß das Einstechrohr ungefähr entlang einer der Diagonalen des Behälters in diesen eingeführt wird und die Öffnung des Einstechrohres somit im Bereich einer der Ecken zu liegen kommt, so daß auf diese Art und Weise und durch. eine Ansaugung die Nährflüssigkeit auch bei nach oben weisender Öffnung entnommen werden kann.

Durch die Verwendung eines als Einmalartikel ausgebildeten Behälters ergibt sich ferner der Vorteil, daß ein Umfüllen der Nährflüssigkeit in andere gesonderte Behälter für das Ernährungssystem entfallen kann, was Arbeits-und Materialkosten einspart. Dabei können direkt die Einmal-Behälter verwendet werden, in denen die Nährflüssigkeit verkauft wird, obwohl deren Öffnung in aller Regel lediglich mit einer einfachen Cellophan-oder Alu-Schicht verschlossen ist, die bei eingeführtem Einstechrohr keinerlei Dichtwirkung aufbauen würde. Wie gesagt, ist dies jedoch bei der erfindungsgemäßen Anordnung nicht erforderlich, da der Behälter ohnehin mit nach oben weisender Öffnung angeordnet wird.

Die Unteransprüche haben vorteilhafte Weiterbildungen der Erfindung zum Inhalt.

Auf besonders einfache Art und Weise ist die geneigte Aufhängung des Behälters durch eine gemäß Anspruch 2 ausgebildete Aufhängevorrichtung erreichbar, bei der eine Haltevorrichtung vorgesehen ist, in die der Behälter eingesetzt wird und an dem ein Aufhängeteil befestigt ist, das beispielsweise in Form einer Platte oder Leiste ausgebildet sein kann. Dieses Aufhängeteil ist in einem Winkel zur Haltevorrichtung angeordnet, so daß der Behälter in aufgehängtem Zustand durch Schwerkraftwirkung automatisch in die gewünschte gekippte Lage überführt wird, ohne daß weitere Maßnahmen erforderlich wären. Die Größe des Winkels hängt vor allem von der Behälterform und -größe ab. Um Anpassungsmöglichkeiten an unterschiedliche Behälterarten bei Verwendung einer Aufhängevorrichtung zu ermöglichen, wäre es denkbar, das Aufhängeteil beweglich am Halterahmen zu befestigen, so daß eine Einstellung des jeweils gewünschten Winkels möglich ist.

Die Anordnung des Aufhängeteils an der Haltevorrichtung kann beispielsweise derart erfolgen, daß die Längsachse des Aufhängeteils unter einem Winkel zur Längsachse der Haltevorrichtung verläuft, insbesondere das Aufhängeteil und die Haltevorrichtung einen Winkel $\alpha$ von 140° ± 10° einschließen. Die Anordnung kann ferner derart erfolgen, daß die Längsachse des Aufnahmeteils und die Querachse der Haltevorrichtung unter einem Winkel von ungleich 90° zueinander verlaufen, insbesondere die Längsachse des Aufnahmeteils und die Querachse der Haltevorrichtung einen Winkel $\beta$ von 70° + 10° einschließen. Es liegt gleichfalls im Rahmen der Erfindung, die Anordnung der beiden Teile so vorzunehmen, daß diese sowohl unter dem genannten Winkel α als auch unter dem genannten Winkel β angeordnet sind.

Eine besonders einfach herzustellende Ausführungsform für die Haltevorrichtung ist ein Halterahmen aus drei Platten, die der jeweiligen Form des aufzunehmenden Behälters angepaßt werden können, so daß ein Aufnahmeraum für den Behälter entsteht. Als Materialien kommen beispielsweise Kunststoffe oder Leichtmetalle in Frage, wobei die Sicherung des Behälters in dem Aufnahmeraum noch durch das Vorsehen von wenigstens zwei paarweise angeordneten Haltelaschen erhöht werden kann, die jeweils an einer Platte angeordnet sind und den Behälter an den Seiten umgreifen, die nicht von den Platten gehalten werden.

Weist der Behälter - auf die gekippte Position des Behälters bezogen - im Bereich seiner oberen Kante eine vom Behälter wegweisende Lasche auf, so genügt es, wenn die Haltevorrichtung als Platte ausgebildet ist, mit einem der Abmessung der Lasche angepaßten Schlitz zur Aufnahme der Lasche. Zweckmäßig weist die Platte einen ersten vom Aufhängeteil weggerichteten Teil und einen zweiten, sich an den ersten Teil anschließenden, zum Aufhängeteil gerichteten, zweiten Teil auf, wobei im Bereich der Verbindung der beiden Teile der Schlitz angeordnet ist. Der Behälter mit dem Schlitz wird durch die beiden in etwa parallel zueinander angeordneten Teile sicher gehalten. Zweckmäßig besitzt das freie Ende des zweiten Teils eine der Abmessung der Lasche des Behälters angepaßte Ausnehmung. Hierdurch können auch Behälter mit Laschen geringerer Abmessung von der Platte sicher fixiert werden, indem die Lasche seitlich an der äußeren Begrenzung der Ausnehmung anliegt. Die Sicherung des Behälters kann zusätzlich durch seitlich der Platte angeordnete Haltelaschen erhöht werden. Beim Entleeren des Behälters wird dieser aufgrund des sich verändernden Gesamtschwerpunktes von Behälter und Aufhängevorrichtung um den Aufhängepunkt des Aufhängeteils geringfügig ver - schwenkt.

Eine vorteilhafte Ausführungsform der Erfindung sieht vor, in der Aufhängevorrichtung einen Zeiger schwenkbar gelagert anzuordnen, mit dem aufgrund der veränderlichen Winkellage der Aufhängevorrichtung das Behältergewicht bestimmbar ist. Der Zeiger ist zweckmäßig im Bereich seines dem Aufhängepunkt zugewandten Endes hakenförmig ausgebildet, das Ende durchsetzt das Loch zum Aufhängen der Aufhängevorrichtung, das insbesondere eine zur Haltevorrichtung gerichtete Kerbe aufweist. Um den Fußpunkt der Kerbe kann sich der Zeiger der Schwenkbewegung der Aufhängevorrichtung und des Behälters beim Entleeren des Behälters anpassen und so das Behältergewicht indirekt anzeigen. Das kerbförmig ausgebildete Loch beinhaltet zudem den Vorteil, daß bei Weglassen des Zeigers zum Anzeigen des Behältergewichts ein Schlauchende in die Kerbe eingeklemmt werden kann. Alternativ kann das Behältergewicht dadurch bestimmt werden, daß die Aufhängevorrichtung ein federbelastetes Glied und eine Skala aufweist.

Um das Ansaugen der Nährflüssigkeit aus dem Behälter zu erleichtern, kann im Schlauchsystem eine pumpfähige Tropfkammer angeordnet sein, mittels der die Nährflüs sigkeit aus dem Behälter angesaugt werden kann, wonach die aus der Tropfkammer durch Schwerkraft direkt oder gegebenenfalls über eine zwischengeschaltete Schlauchpumpe dem Patienten zugeführt werden kann.

Als besonders bevorzugte Ausbildung für die Tropfkammer ist eine solche aus elastisch zusammendrückbarem Material vorgesehen, mit welcher zunächst Luft in den Behälter gepumpt werden kann, wonach bei sich wieder ausdehnender Tropfkammer die Flüssigkeitsansaugung stattfindet.

Um das Ansaugen weiterhin zu erleichtern, kann nach der Tropfkammer eine Schlauchklemme im Schlauchleitungssystem angeordnet sein, die das von der Tropfkammer kommende Schlauchende während des Ansaugvorgangs absperrt.

Weitere Einzelheiten, Merkmale und Vorteile der Erfindung ergeben sich aus nachfolgender Beschreibung mehrerer Ausführungsbeispiele anhand der Zeichnung.

Es zeigt:

Fig. 1 eine schematisch leicht vereinfachte Darstellung eines erfindungsgemäßen Ernährungssystems mit einer ersten Ausführungsform einer Aufhängevorrichtung,

Fig. 2 eine der Fig. 1 entsprechende Darstellung eines Längsschnittes durch ein Einsteckrohr des erfindungsgemäßen Systems,

Fig. 3 eine der Fig. 1 entsprechende Darstellung des Ernährungssystems während des Anstechens eines Behälters des erfindungsgemäßen Systems,

Fig. 4 eine zweite Ausführungsform einer Aufhängevorrichtung für das erfindungsgemäße System,

Fig. 5 die Aufhängevorrichtung in der Ansicht X nach Fig. 4,

Fig. 6 eine dritte Ausführungsform einer Aufhängevorrichtung für das erfindungsgemäße System,

Fig. 7 den Aufhängebereich der Aufhängevorrichtung der Ansicht Y nach Fig. 6,

Fig. 8 eine vierte Ausführungsform einer Aufhängevorrichtung für das erfindungsgemäße System,

Fig. 9 eine fünfte Ausführungsform einer Aufhängevorrichtung für das erfindungsgemäße System,

Fig.10 den Aufhängebereich der Aufhängevorrichtung der Ansicht Z nach Fig. 9 und

Fig.11 einen Behälter zur Anwendung im erfindungsgemäßen Ernährungssystem.

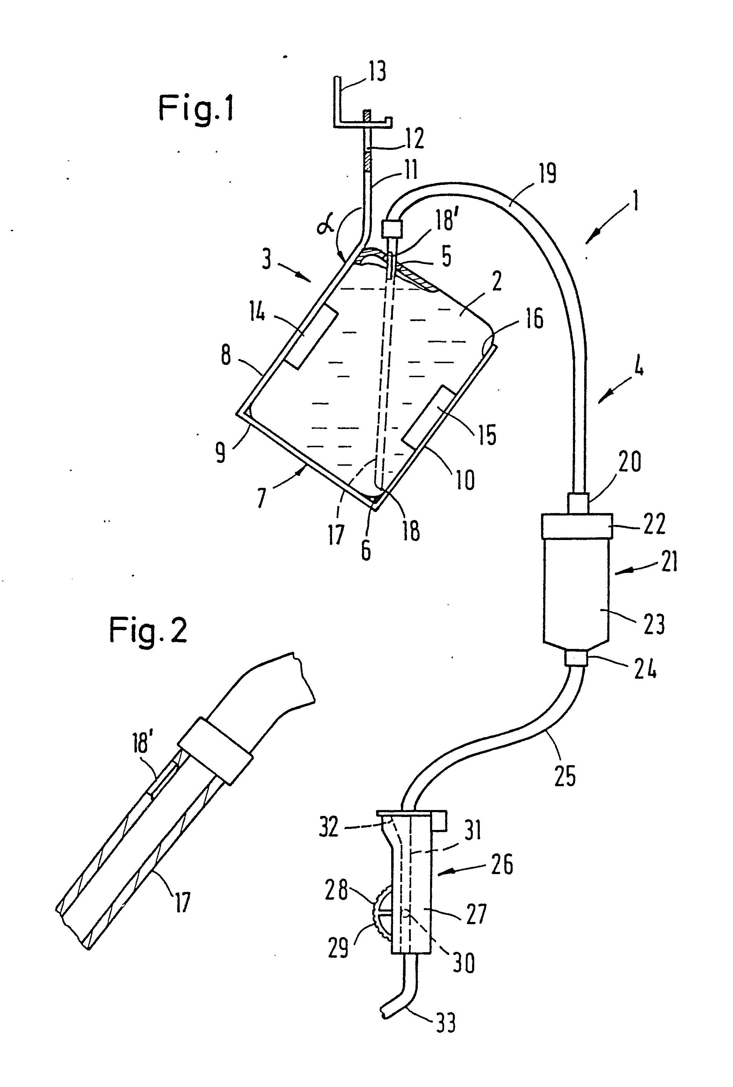

In den Fig. 1 - 3 ist ein Ernährungssystem 1 dargestellt, das einen vorzugsweise als Einmalartikel ausgebildeten Behälter 2 zur Aufnahme der Nahrung, vorzugsweise Flüssignahrung, eine Aufhängevorrichtung 3 zum Aufhängen des Behälters 2 und ein Schlauchleitungssystem 4 zur Zuführung der Nahrung zu einem in der Zeichnung nicht näher dargestellten Patienten aufweist.

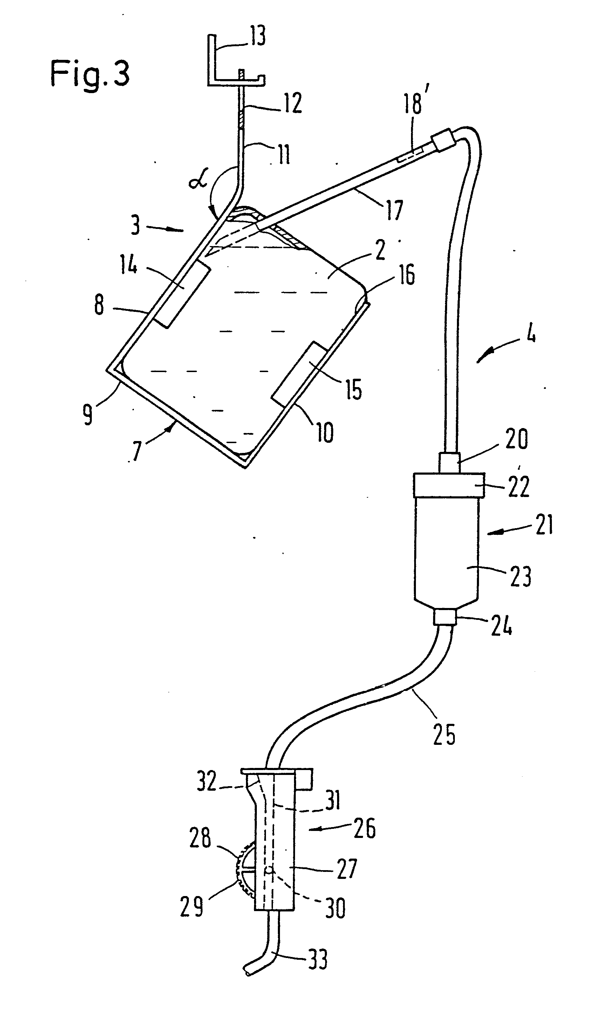

Wie aus den Figuren 1 und 3 ersichtlich ist, ist der Behälter 2 in der Aufhängevorrichtung 3 derart angeordnet, daß er im aufgehängten Zustand einen Winkel zur Horizontalen einnimmt, so daß im Beispielsfalle eine Ecke 6 des Behälters 2 den tiefsten Punkt bildet. Hierfür ist die Aufhängevorrichtung 3 derart ausgebildet, daß der Behälter 2 allein durch Schwerkraft im aufgehängten Zustand die in den genannten Figuren dargestellte Position einnimmt. Die Aufhängevorrichtung weist zu diesem Zweck einen Halterahmen 7 auf, der bei der dargestellten Ausführungsform drei Platten 8, 9 und 10 aufweist, die jeweils im wesentlichen im rechten Winkel zueinander angeordnet sind. Die Platten 8, 9 und 10 können hierbei entweder einstückig aneinander angeformt oder auf andere Art und Weise, wie beispielsweise eine Schweißung oder Klebung, miteinander verbunden sein. Am Halterahmen 7 ist ein Aufhängeteil 11 befestigt. Die Längsachse 50 des Aufhängeteils 11 schließt mit der Längsachse 51 der Platte 8 einen Winkel α ein, der bei der dargestellten Ausführungsform so gewählt ist, daß der rechteckige bzw. quaderförmige Behälter 2 die in den genannten Figuren dargestellte Position einnimmt, in der die Öffnung 5 nach oben weist und eine der Ecken, im dargestellten Falle die Ecke 6, den tiefsten Punkt des Behälters 2 bildet. In aller Regel beträgt der Winkel etwa 140° ± 10°. Die in den Figuren nicht näher dargestellte Querachse der Platte 8 verläuft unter einem rechten Winkel zur Längsachse 50 des Aufhängeteils 11.

Das Aufhängeteil 11 ist im Beispielsfalle einstückig am Halterahmen 7 befestigt, könnte jedoch auch über ein selbsthemmendes Scharnier am Halterahmen 7 angelenkt werden, so daß eine Einstellung des Winkels α in Abhängigkeit von der gewünschten Neigung des Behälters 2 möglich ist. Das Aufhängeteil 11, das als Leiste oder Platte ausgebildet sein kann, weist eine Ausnehmung 12 auf, in die ein Teil einer ortsfesten Befestigung eingeführt werden kann, wie dies in den Figuren 1 und 3 durch einen Haken 13 angedeutet ist.

Wie ferner aus diesen Figuren ersichtlich ist, sind im Beispielsfalle an den Platten 8 und 10 jeweils zwei Haltelaschen angeordnet, von denen nur die vorderen Haltelaschen 14 bzw. 15 zu sehen sind. Diese dienen dazu, den Behälter 2 in einem von den Platten 8, 9 und 10 begrenzten Aufnahmeraum 16 sicher zu halten.

Das Schlauchsystem 4 weist bei dem erfindungsgemäßen Ernährungssystem 1 ein Einsteckrohr 17 auf, das das im Behälter 2 angeordnete Ende des Schlauchsystems 4 bildet und das eine Einstechspitze 18 und eine am Umfang und im oberen Bereich des Einsteckrohres 17 angeordnete und sich parallel zu dessen Längserstreckung erstreckende Belüftungskerbe 18' aufweist, deren Anordnung und Ausbildung im einzelnen der Fig. 2 zu entnehmen ist. Das Einsteckrohr 17 wird durch den Verschluß der Öffnung 5 in den Behälter 2 eingeführt und an dessen tiefster Stelle, wie im Beispielsfalle der Ecke 6, angeordnet, so daß die gesamte im Behälter 2 enthaltene Nährflüssigkeit trotz nach oben weisender Öffnung 5 entnommen werden kann.

Das Einsteckrohr 17, welches auch als selbständiges Teil ausgebildet sein kann, ist außerhalb des Behälters 2 mit einem Leitungsstück 19 aufsteckbar verbunden, das über einen Anschluß 20 mit einer Tropfkammer 21 in Strömungsverbindung steht. Die Tropfkammer 21 weist einen Deckel 22, einen im Beispielsfalle aus elastisch zusammendrückbarem Material bestehenden zylindrischen Hauptkörper 23 und einen weiteren Anschlußzapfen 24 auf. An diesen Zapfen 24 schließt sich ein weiteres Leitungsstück 25 an, das in einer Schlauchklemme 26 mündet. Diese Schlauchklemme 26 weist ein Führungsteil 27 für ein Klemmrad 28 auf, das eine gerippte Oberfläche 29 aufweist. Das Klemmrad 28 ist mit einem nicht näher dargestellten durchgehenden und über die Seitenbereiche des Klemmrades 28 hinausreichenden Zapfen versehen, der zwei über den Seitenbereich des Klemmrades 28 hinausreichende Enden aufweist, von denen in der Figur ein Ende 30 in Draufsicht angedeutet ist. Dieses Ende 30, wie auch das diesem gegenüberliegende, gleitet in einer über die gesamte Länge der Schlauchklemme sich erstreckende Führungsausnehmung 31, die einen erweiterten Bereich 32 aufweist. Soll der Querschnitt des Leitungsstückes 25 freigegeben werden, wird das Klemmrad 28 in Richtung auf den erweiterten Bereich 32 geführt, wodurch es sich vom Schlauchstück 25 entfernt und so den Querschnitt freigibt. In der entgegengesetzten Richtung klemmt das Klemmrad 28 die Schlauchwände zusammen und sperrt auf diese Art und Weise den Schlauchquerschnitt vollständig ab. Diese Stellung ist in der Figur angedeutet.

Im Anschluß an die Schlauchklemme 26, also in dem von der Schlauchklemme 26 abgehenden Leitungsstück 33, das zum Patienten führt, kann ergänzend eine in der Figur nicht näher dargestellte Schlauchpumpe angeordnet sein.

Soll nun einem Patienten Nährlösung zugeführt werden, wird zunächst der Behälter 2 in der Aufhängevorrichtung 3 angeordnet, wonach diese am Haken 13 aufgehängt wird, wodurch sich aufgrund der Schwerkraftwirkung die in der Fig.1 dargestellte Stellung des Behälters 2 mit nach oben weisender Öffnung 5 und einer zur Horizontalen entstehenden Neigung einstellt. Danach wird das Einsteckrohr 17 durch den durchstechbaren Verschluß der Öffnung 5 zunächst in einer ersten Phase des Anstechvorganges gemäß Fig. 3 zu ca. einem Drittel in den Behälter 2 eingestochen. In diesem in Fig. 3 im einzelnen dargestellten Zustand liegt das Einsteckrohr 17 dicht an der Einstichöffnung 5 an, so daß keine Flüssigkeit austreten kann. In diesem Zustand wird der Pumpvorgang mittels der Tropfkammer 21 begonnen, bei welcher die Dosier-bzw. Schlauchklemme 26 geschlossen ist. Zu Beginn wird das Einsteckrohr 17 deshalb nur bis zu ca. einem Drittel in den Behälter 2 eingeführt, da dieser ohne Luftinhalt vollkommen mit Flüssigkeit gefüllt ist, so daß bei einem vollständigen Einführen des Einsteckrohres 17, bei welchem die Belüftungskerbe 18' in der Öffnung 5 angeordnet wäre, Flüssigkeit bzw. Nahrung aus dem Behälter 2 auslaufen könnte.

Nach dieser ersten Phase wird in einer gemäß Fig. 1 dargestellten zweiten Phase das Einsteckrohr, im Beispiels falle ungefähr entlang der Diagonalen des Behälters 2, in diesen eingeführt, bis die Öffnung des Einsteckrohrs 17 die tiefste Stelle 6 des Behälters erreicht hat. In dieser Stellung des Einsteckrohres ist die Belüftungskerbe 18' in der Öffnung 5 angeordnet, so daß die Belüftungskerbe 18' ihre Funktion, nämlich das Belüften des Behälters 2, übernehmen kann, ohne daß Flüssigkeit aus dem Behälter 2 auslaufen kann. In diesem Zustand wird die Schlauchklemme 26 geöffnet.

Danach kann die Nährlösung weiter aus dem Behälter abgesaugt werden, wozu im Beispielsfalle die zwischen der Schlauchklemme 26 und dem Behälter 2 angeordnete Tropfkammer 21, die zudem unterhalb der tiefsten Stelle des Behälters 2 angeordnet ist, zusammengedrückt wird, was aufgrund des elastisch zusammendrückbaren Materials möglich ist. Dabei wird zunächst eventuell noch im Leitungsstück 19 befindliche Luft in den Behälter 2 geführt, da zuvor die Schlauchklemme 27 abgesperrt war. Entspannt sich die Tropfkammer nach dem Zusammendrücken wieder, wird Flüssigkeit aus dem Behälter 2 in die Tropfkammer 21 angesaugt, so daß eine durchgehende Flüssigkeitssäule von der tiefsten Stelle des Behälters 2 bis in die Tropfkammer 21 entsteht, so daß nach dem anfänglichen Ansaugen durch Zusammendrücken der Tropfkammer 21, nunmehr ständig Flüssigkeit aus dem Behälter 2 abgesaugt wird. Diese Flüssigkeit wird nach Durchlaufen der Tropfkammer 21 über die geöffnete Schlauchklemme 26 entweder durch Schwerkrafteinwirkung oder durch eine zusätzlich zwischengeschaltete Pumpe dem Patienten zugeführt.

Wie die Fig. 1 zeigt, ist bei dieser Anordnung ein Undichtwerden des Behälters 2 durch ein unerwünschtes Austropfen von Flüssigkeit durch die Öffnung 5 und entlang des Außenumfangs des Einsteckrohrs 17 wirksam verhindert, da es zu einem derartigen Heraustropfen aufgrund der nach oben weisenden Anordnung der Öffnung 5 auch ohne das Vorsehen von besonderen Dichteinrichtungen nicht kommen kann. Dabei wird durch die geneigte Anordnung des Behälters 2 trotzdem ein vollständiges Entleeren desselben sichergestellt.

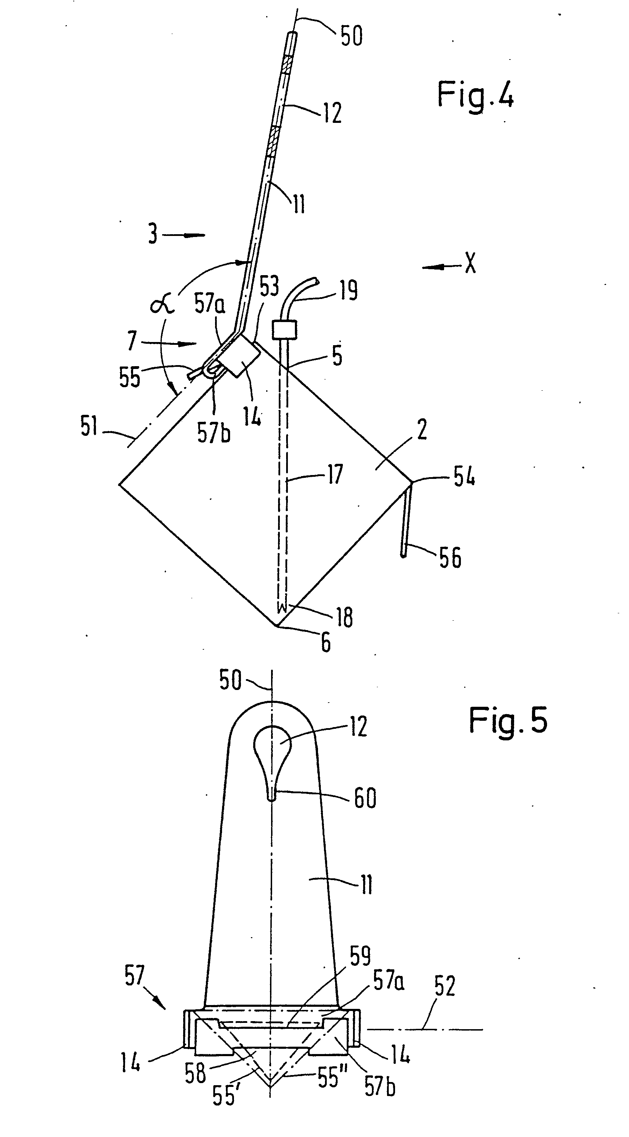

In den Fig. 4 - 10 sind weitere Ausführungsformen der Aufhängevorrichtung 3 zum Aufhängen des Behälters 2 gezeigt. Mit der Aufhängevorrichtung 3 gemäß der Darstellung der Fig. 1 - 3 in ihrer Wirkung übereinstimmende Teile wurden mit gleichen Bezugsziffern bezeichnet.

Auf die Darstellung des vollständigen Ernährungssystems wurde verzichtet, um Wiederholungen zu vermeiden.

Die vorstehenden Ausführungen zu dem Ernährungssystem sind entsprechend auf die nachfolgend geschilderten Ausführungsformen zu lesen.

Die Aufhängevorrichtungen gemäß der Fig. 4 - 10 dienen der Aufnahme das als Einmalartikel ausgebildeten Behälters 2, der rechteckförmig ausgebildet ist, jedoch im Bereich seiner beiden oberen, gegenüberliegenden Kanten 53 und 54 dreieckförmige Laschen 55 und 56 aufweist. Die Befestigung des Behälters 2 an der Aufhängevorrichtung 3 erfolgt dabei in noch näher zu beschreibender Art und Weise im Bereich der Lasche 55.

Bei der Ausführungsform nach den Fig. 4 und 5 ist die Haltevorrichtung 7 als Platte 57 ausgebildet, an der das Aufhängeteil 11 befestigt ist. Die Längsachse 50 des Aufhängeteils 11 schließt mit der Längsachse 51 den bereits bei der vorhergehenden Ausführungsform erörterten Winkel α ein. Die Platte 57 besteht aus einem ersten, vom Aufhängeteil 11 weggerichteten Teil 57a und einem zweiten, sich an den ersten Teil 57a anschließenden zum Aufhängeteil 11 gerichteten zweiten Teil 57b, wobei im Bereich der Verbindung der beiden Teile 57a, 57b ein in Querrichtung der Teile verlaufender Schlitz 58 angeordnet ist. Die Befestigung des Behälters 2 am Aufhängeteil 11 erfolgt, indem der Behälter 2 mit dessen Lasche 55 von oben zwischen die beiden Teile 57a und 57b der Platte 57 eingeführt wird und dabei das spitze Ende der Lasche 55 den Schlitz 58 durchsetzt. Die beiden Teile 57a und 57b sind dabei derart beabstandet, daß die Lasche 55 gerade zwischen diesen hindurchgeschoben werden kann und durch das Eigengewicht des Behälters 2 zwischen den Teilen 57a und 57b geklemmt wird. In der Fig. 5 sind Positionen verdeutlicht, die eine kleine Lasche 55' bzw. eine große Lasche 55" in eingeschobenem Zustand einnehmen würden. Damit der Behälter 2 sicher von der Haltevorrichtung 7 gehalten wird, sind beidseitig der Platte 57 Haltelaschen 14 angeordnet. Das freie Ende des Teiles 57b weist eine Ausnehmung 59 auf, die der Abmessung der kleineren Lasche 55' angepaßt ist, so daß diese Lasche 55' zwischen den seitlichen Begrenzungen der Ausnehmung 59 sicher gehalten ist.

Wie der Darstellung der Fig. 5 zu entnehmen ist, verläuft die Längsachse 50 des Aufnahmeteils 11 unter einem rechten Winkel zur Querachse 52 der Platte 57. Die Ausnehmung 12 im Aufhängeteil 11 besitzt eine zur Platte 57 gerichtete Kerbe, in die nach dem Entfernen des Einstechrohres 17 aus dem Behälter 2 das Leitungsstück 19 eingeklemmt werden kann. Im Unterschied zu der Ausbildung des Einstechrohres 17 nach den Fig. 1 und 3 ist das Einstechrohr 17, wie der Fig. 4 zu entnehmen ist, an seinem unteren Ende 18 kerbförmig ausgebildet, wodurch ein Verstopfen des Einstechrohres 17 wirksam verhindert werden kann.

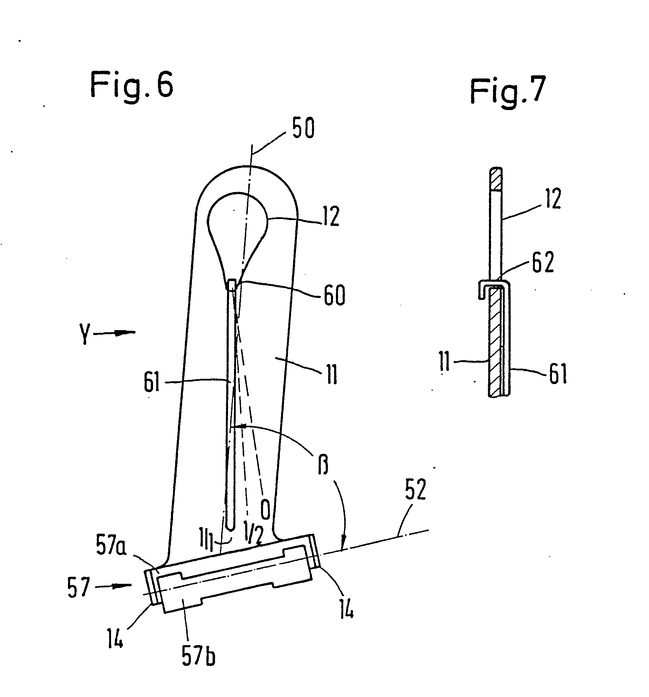

Die Ausführungsform nach den Fig. 6 und 7 stimmt im wesentlichen mit der Ausführungsform gemäß den Fig. 4 und 5 überein. Im Unterschied verläuft, wie der Fig. 6 zu entnehmen ist, die Längsache 50 des Aufnahmeteils 11 bezüglich der Querachse 52 der Platte 57 unter einem Winkel β, der ungleich einem rechten Winkel ist, der insbesondere 70° ± 10° beträgt. Der Winkel α entspricht dabei dem Winkel α der Ausführungsform nach der Fig. 4 bzw. 5, es ist jedoch gleichfalls möglich, einen Winkel α von 180° vorzusehen.

Wie der Fig. 6 ferner zu entnehmen ist, ist die Ausnehmung 12 außermittig angeordnet, d.h. sie befindet sich auf der Seite des Aufhängeteils 11, das mit der Querachse 52 einen stumpfen Winkel einschließt. Die Ausnehmung 12 besitzt gleichfalls eine Kerbe 60, in diese ist ein Zeiger 61 eingehängt. Mittels des Zeigers 61 kann das Behältergewicht bestimmt werden, da das Aufhängeteil 11 aufgrund des sich während des Entleerens des Behälters 2 verändernden Gesamtschwerpunktes von Behälter 2 und Aufhängeteil 11 eine Schwenkbewegung um dessen Aufhängepunkt vollführt. Markierungen im Bereich des unteren Endes des Zeigers geben einen Aufschluß, wieviel Menge sich im Behälter noch befindet. In der Fig. 6 ist dargestellt, welche Position der Zeiger 61 bezüglich des Aufhängeteils 11 einnimmt, wenn der Behälter zu 1/1, 1/2 gefüllt ist oder leer ist. Es versteht sich, daß die mit strichlierten Linien dargestellten Zwischenstellungen nur die Position des Zeigers zum Aufhängeteil 11 beschreiben, während in Realität natürlich das Aufhängeteil 11 un dem Aufhängepunkt des Aufhängeteils verschwenkt wird. Bei Versuchen hat sich gezeigt, daß sich das Behältergewicht durch diese einfache Zeigeranordnung mit einer Genauigkeit von etwa 10 % bestimmen läßt. Besonders einfach ist die Zeigerariordnung gestaltet, wenn der Zeiger 61 im Bereich der Ausnehmung 12 mit einem Haken 62 versehen ist, der einfach in die Kerbe 60 eingehängt wird.

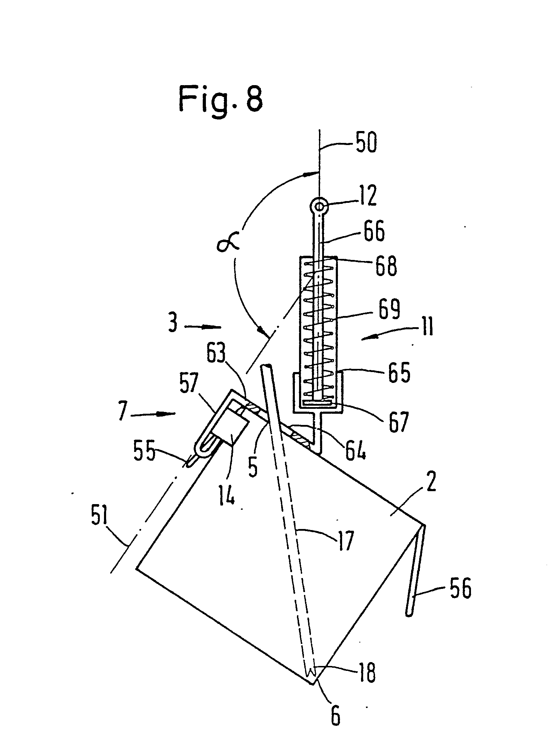

Bei der Ausführungsform nach Fig. 8 weist die Haltevorrichtung 7 im Anschluß an die Platte 57 eine senkrecht zu dieser verlaufende Deckplatte 63 auf. Mit dem freien Ende der Deckplatte 63 ist das Aufhängeteil 11 befestigt, die Längsachse 50 des Aufhängeteils 11 schließt wiederum mit der Längsachse 51 der Platte 57 einen Winkel α von etwa 140° + 10° ein. Der Winkel β ist in der Fig. 8 nicht dargestellt, dieser kann wie bei der Ausführungsform nach Fig. 4 bzw. 5 90° betragen oder wie bei der Ausführungsform nach Fig. 6 und 7 70° ± 10°. In der Deckplatte 63 ist eine Ausnehmung 64 zum Durchstechen des Einstechrohres 17 vorgesehen. Das Aufhängeteil 11 weist einen Zylinder 65 auf, in dem ein mit einer Kolbenstange 66 versehener Kolben 67 geführt ist, zwischen dem Zylinderdeckel 68 und dem Kolben 67 befindet sich eine Druckfeder 69. Das dem Kolben 67 abgewandte Ende der Kolbenstange 66 ist mit der Ausnehmung 12 versehen. Die Ausbildung des Aufhängeteils 12 in Art eines federbelasteten Bauteils bietet eine weitere Möglichkeit, die Entleerung des Behälters 2 optisch anzuzeigen. So kann beispielsweise auf der Kolbenstange 66 eine Markierung angeordnet sein, die unmittelbar Aufschluß über die verbleibende Gewichtsmenge im Behälter 2 gibt. Es ist gleichfalls denkbar, die Bewegung des Kolbens 67 auf ein nicht näher dargestelltes Steuerelement zu übertragen, welches bei einem bestimmten Kolbenweg das Schlauchsystem blockiert, so daß dem Patienten keine Nahrung mehr zugeführt wird.

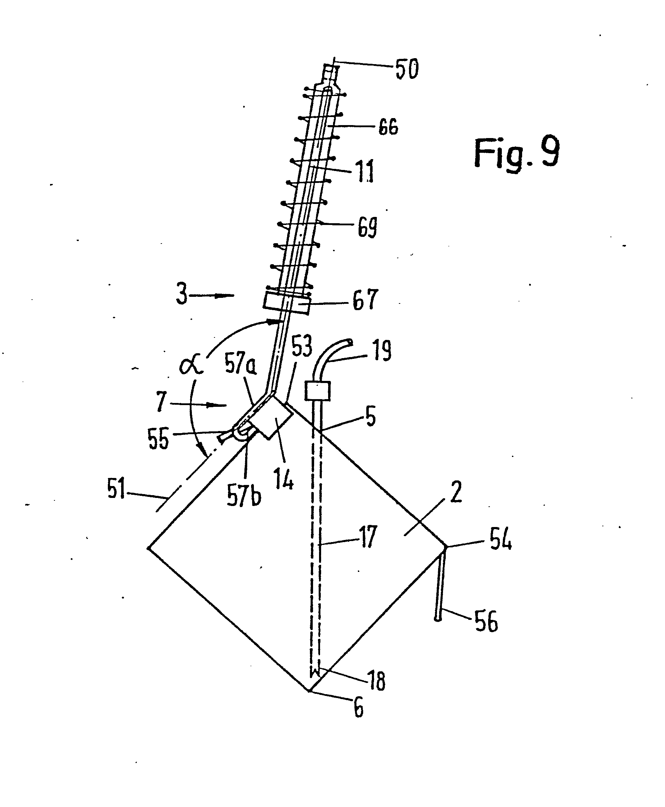

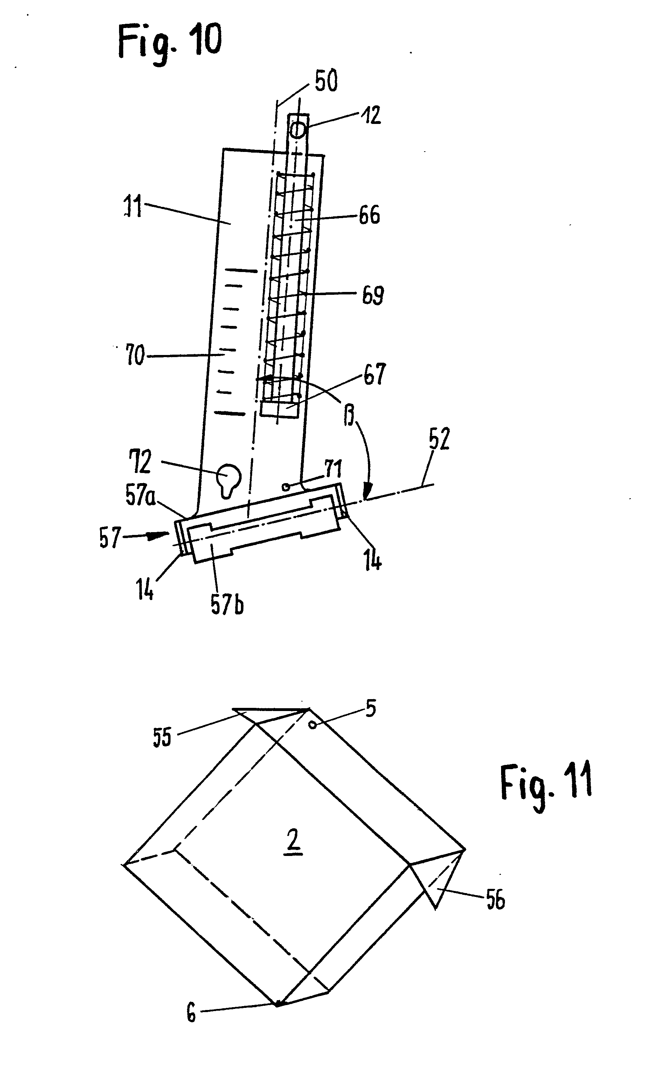

Die Ausführungsform nach den Fig. 9 und 10 stimmt im wesentlichen mit der Ausführungsform gemäß Fig. 6 überein. Im Unterschied dazu ist, wie Fig. 9 zu entnehmen ist, statt des Zeigers 61 ein federbelastetes Glied ähnlich dem aus Fig. 8 am Aufnahmeteil 11 angeordnet. Dieses federbelastete Glied besteht aus einer Kolbenstange 66, einem Kolben 67 und einer Druckfeder 69. Der Kolben 67 ist bewegbar im Aufnahmeteil 11 und führt die Druckfeder 69, die am anderen Ende vom Aufnahmeteil 11 abge stützt wird. Die federbelastete Aufnahmevorrichtung wird dabei mit der Ausnehmung 12 ortsfest befestigt.

Aus Fig. 10 ist ersichtlich, daß das federbelastete Glied 66; 67; 69 außermittig an dem Aufnahmeteil 11 angeordnet ist, wobei die Einhaltung des Winkels α von ungefähr 140° gewährleistet ist. Unmittelbar neben dem federbelasteten Glied 66, 67, 69, das als Federwaage ausgebildet ist, ist eine Skala 70 angebracht, auf der mit Hilfe einer nicht näher dargestellten Markierung auf dem Kolben 67 das Behältergewicht bzw. die Behälterfüllmenge bei entsprechender Verschiebung des Kolbens 67 durch Entleerung des Behälters 2 abgelesen werden kann.

Das Aufnahmeteil 11 besitzt weiterhin eine Markierung 71, die deutlich macht, daß die Öffnung 5 des Behälters 2 in unmittelbarer Nähe dieser Markierung 71 fixiert sein muß. Damit werden unsachgemäße Plazierungen des Behälters 2 ausgeschlossen.

Ein kerbförmiger Durchbruch 72 ist am Aufnahmeteil 11 vorgesehen, um den Schlauch 19 nach Austritt vom Einsteckrohr 17 aus dem Behälter 2 verrutschfest zu verklemmen bzw. als Aufhängeelement zu dienen.

Fig. 11 zeigt in einer perspektivischen Darstellung den im erfindungsgemäßen Ernährungssystem verwendeten Behälter 2. Dieser ist vorzugsweise als Einmalartikel im Bedarfsfall rechteckförmig ausgebildet und weist eine, beispielsweise mit Folie, verschlossene Öffnung 5 auf, in die das Einsteckrohr 17 eingeführt wird. Es sind deutlicher die Verschlußlaschen 55 und 56 erkennbar. Der Behälter 2 ist so in die Platte 57 bzw. den Halterahmen 7 einsetzbar, daß die Öffnung 5 in unmittelbarer Nähe der Markierung 71 gebracht und die Verschlußlasche 55 in den vorgesehenen Schlitz 58 eingesetzt wird. Nach der ortsfesten Befestigung der Aufhängevorrichtung 3 hat der Behälter 2 an der Ecke 6 seinen tiefsten Punkt.

Derartige Behälter 2 bestehen üblicherweise aus gewachster Pappe und weisen in ihrem Innenraum eine flüssigkeitsundurchlässige Schicht aus einer Kunststoff-oder Aluminiumfolie auf. Sie werden üblicherweise zur Aufbewahrung von Milch, Fruchtsäften, Flüssignahrung und dergl. verwendet.

**Ansprüche**

1. Ernährungssystem für die enterale Ernährung mit einem Behälter zur Aufnahme der Nahrung, der eine mit einem durchstechbaren Verschluß versehene oder aufreißbare Öffnung aufweist,
mit einer Aufhängevorrichtung für den Behälter und mit einem Schlauchleitungssystem, dessen eines Ende im Behälter angeordnet ist und dessen anderes Ende der zu ernährenden Person zuführbar ist, **dadurch gekennzeichnet, daß** der Behälter (2) mit seiner Öffnung (5) nach oben weisend und in einem Winkel zur Horizontalen geneigt aufhängbar ist,
daß das im Behälter (2) angeordnete Ende des Schlauchsystems (4) als Einsteckrohr (17) ausgebildet ist, dessen Öffnung (18) an der tiefsten Stelle (6) des Behälters (2) anordenbar ist und daß der Behälter (2) als Einmalartikel ausgebildet ist.

2. Ernährungssystem nach Anspruch 1, **dadurch gekennzeichnet, daß** die Aufhängevorrichtung (3) eine Haltevorrichtung (7) für den Behälter (2) und ein an diesem befestigtes Aufhängeteil (11) aufweist, das in einem solchen Winkel (α, β) zur Haltevorrichtung (7) angeordnet ist, daß der Behälter (2) in aufgehängtem Zustand die gekippte Lage einnimmt, wobei die Längsachse (50) des Aufhängeteils (11) unter einem Winkel zur Längsachse (51) der Haltevorrichtung (7) verläuft, insbesondere das Aufhängeteil (11) und die Haltevorrichtung (7) einen Winkel α von 140° ± 10° einschließen und die Längsachse (50) des Aufhängeteils (11) und die Querachse (52) der Haltevorrichtung (7) unter einem Winkel von ungleich 90° zueinander verlaufen, insbesondere die Längsachse (50) des Aufnahmeteils (11) und die Querachse (52) der Haltevorrichtung (7) einen Winkel β von 70° ± 10° einschließen.

3. Ernährungssystem nach einem der Ansprüche 1-2, **dadurch gekennzeichnet, daß** die Haltevorrichtung als Halterahmen (7) ausgebildet ist, der drei Platten (8, 9, 10) aufweist, die einen der Form des aufzunehmenden Behälters (2) angepaßten Aufnahmeraum (16) begrenzen, wobei an wenigstens zwei der Platten (8, 9, 10) paarweise Haltelaschen (14, 15) angeordnet sind.

4. Ernährungssystem nach einem der Ansprüche 1-3, **dadurch gekennzeichnet ,** daß der Behälter (2) im Bereich seiner oberen Kante (53) eine vom Behälter (2) wegweisende Lasche (55) aufweist, und die Haltevorrichtung (7) als Platte (57) ausgebildet ist, mit einem der Abmessung der Lasche (55) angepaßten Schlitz (58) zur Aufnahme der Lasche (55), wobei die Platte (57), an der seitlich Haltelaschen (14) angeordnet sind, einen ersten vom Aufhängeteil (11) weggerichteten Teil (57a) und einen zweiten, sich an den ersten Teil (57a) anschließenden, zum Aufhängeteil (11) gerichteten, zweiten Teil (57b) aufweist, wobei im Bereich der Verbindung der beiden Teile (57a, 57b) der Schlitz (58) angeordnet und das freie Ende des zweiten Teils (57b) eine der Abmessung der Lasche (55) des Behälters (2) angepaßte Ausnehmung (59) aufweist.

5. Ernährungssystem nach einem der Ansprüche 1-4, **dadurch gekennzeichnet,** daß die Aufhängevorrichtung (11) ein federbelastetes Glied (65, 66, 67, 68, 69) und einen Zeiger (61) aufweist, der schwenkbar gelagert ist, mit dem aufgrund der veränderlichen Winkellage der Aufhängevorrichtung (11) mittels einer Skala (70) das Behältergewicht bestimmbar ist.

6. Ernährungssystem nach einem der Ansprüche 1-5, **dadurch gekennzeichnet,** daß die Aufhängevorrichtung (11) eine Markierung (71) zur Lagebestimmung der Öffnung (5) des Behälters (2), eine Ausnehmung (12) zum Aufhängen der Aufhängevorrichtung (11) aufweist und daß der Aufhängepunkt der Aufhängevorrichtung (11) bezüglich der Längsachse (50) des Aufhängeteils (11) außermittig angeordnet ist.

7. Ernährungssystem nach einem der Ansprüche 1-6, **dadurch gekennzeichnet ,** daß im Anschluß an das Einsteckrohr (17) im Schlauchsystem (4) eine pumpfähige Tropfkammer (21) angeordnet ist, die aus einem elastisch zusammendrückbaren Material besteht und daß nach der Tropfkammer (21) im Schlauchsystem (4) eine Schlauchklemme angeordnet und das Schlauchsystem (4) an eine Schlauchpumpe anschließbar ist.

8. Ernährungssystem nach einem der Ansprüche 1-7, **dadurch gekennzeichnet,** daß das Einsteckrohr (17) eine Belüftungskerbe (18') aufweist.

9. Ernährungssystem nach einem der Ansprüche 1-8, **dadurch gekennzeichnet ,** daß das Einsteckrohr (17) als selbständiges Teil ausgebildet und auf das Ende des Schlauches (19) aufsteckbar ist.

10. Ernährungssystem nach einem der Ansprüche 1-9, **dadurch gekennzeichnet ,** daß die Aufhängevorrichtung einen kerbförmigen Durchbruch (72) zum Einklemmen eines Endes des Schlauches (19) aufweist.

Fig.1

Fig. 2

Fig.3

0 241 595

Fig.4

Fig.5

# Fig. 6

# Fig. 7

# Fig. 8

0 241 595

Fig. 9

## Fig. 10

## Fig. 11

| | **EINSCHLÄGIGE DOKUMENTE** | | EP 86117838.2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - O 119 373 (G.SJÖNELL)  <br> * Fig. 1; Seite 1, Zeilen 2-11; Seite 2, Zeile 15 - Seite 4, Zeile 4 * <br> -- | 1 | A 61 M 1/00 <br> A 61 M 31/00 |
| A | FR - A - 1 282 046 (P.MULTIGNER)  <br> * Fig. 1-3; Seite 1, 2. Spalte, Absätze 5-7 * <br> -- | 1 | |
| A | US - A - 4 432 763 (J.G.MANSCHOT et al.)  <br> * Fig. 1; Zusammenfassung; Spalte 1, Zeilen 6-8; Spalte 1, Zeile 67 - Spalte 4, Zeile 5 * <br> -- | 1 | |
| A | FR - A1 - 2 377 806 (CH.BEFIEUX)  <br> * Gesamt * <br> -- | 1 | RECHERCHIERTE SACHGEBIETE (Int. Cl.4) |
| A | US - A - 4 161 179 (H.J.ABRAMSON)  <br> * Fig. 1-3; Spalte 1, Zeile 60 - Spalte 2, Zeile 5 * <br> ---- | 1 | A 61 M 1/00 <br> A 61 M 5/00 <br> A 61 M 7/00 <br> A 61 M 31/00 <br> B 65 D 33/00 <br> B 65 D 83/00 <br> B 65 D 85/00 |

Der vorliegende Recherchenbericht wurde für alle Patentansprüche erstellt.

| Recherchenort | Abschlußdatum der Recherche | Prüfer |
|---|---|---|
| WIEN | 29-07-1987 | LUDWIG |